# EUROPEAN PATENT APPLICATION

(11) **EP 2 520 934 A1**
(43) Date of publication of application: **07.11.2012**
(21) Application number: 11003654.8
(22) Date of filing: 04.05.2011
(51) Int. Cl.: G01N 33/50, G01N 33/92

(54) **Method for finding active pharmaceutical ingredients for the treatment of deseases associated with increased or decreased cytokine pathway activity**

(71) Applicant: CellMade SAS, 74160 Archamps (FR)
(72) Inventor: Paquaij, Jos, 74160 Archamps (FR); Bronsaer, Ronald, 74160 Archamps (FR)
(74) Representative: Renkema, Jaap

(57) **Abstract**

The invention relates to a method for finding an active pharmaceutical ingredient suitable for the treatment of a disease associated with increased or decreased cytokine pathway activity, comprising the steps of A1) providing a first lipid profile by a) providing a first cell culture medium comprising a first type of cells, b) stimulating the first type of cells, causing the cells to release a pro-inflammatory cytokine, c) bringing a second type of cells having cytokine receptors In a second cell culture medium Into contact with the cell culture medium of the first type of cells causing the cytokine receptors of the second type of cells to respond to the pro-inflammatory cytokine that is released to obtain a cell culture medium that is in contact with the first and the second type of cells, d) adding a component that is potentially suitable for the prevention, treatment or co-treatment of a disease associated with Increased or decreased cytokine pathway activity to the first cell culture medium and/or to the second cell culture medium and/or to the cell culture medium that is in contact with the first and the second type of cells and e) determining the lipid profiles of the second type of cells and/or of the first type of cells and/or of the cell culture medium that is in contact with the first and the second type of cells to obtain the first lipid profile, A2) providing a second lipid profile by performing step A1) except for sub-step d) to obtain the second lipid profile, B) comparing the first lipid profile to the second lipid profile and C) identifying the component that was added in the method that shows a difference between the first lipid profile and the second lipid profile as the active pharmaceutical Ingredient.

## Description

The Invention relates to a method for finding active pharmaceutical ingredients for the treatment of diseases associated with increased or decreased cytokine pathway activity, to active pharmaceutical ingredients thus found and to active pharmaceutical ingredients for use In the treatment of diseases associated with increased or decreased cytokine pathway activity.

Before its discovery in 1984, the tumor necrosis factor (TNF) was the unknown factor that was named after Its ability to regress tumor masses in patients whose tumor had become infected with a bacterial infection. TNF is also known as TNFα, cachectin or differentiation-inducing factor (DIF). TNFα is associated with both 'good' and 'bad' responses. Examples of 'good' TNFα-responses include cell growth and differentiation, inflammatory effects, mediation of immune responses. Examples of 'bad' TNFα-responses include apoptic cell death, necrotic cell death, and its involvement in diseases, for example inflammation diseases, for example acute inflammation, chronic inflammation, psoriasis, periodontitis, rheumatoid arthritis, cardiovascular diseases, osteoarthritis; atherosclerosis, asthma, septic shock, irritable bowel syndrome (IBS), heamorrhagic fever, cachexia, diabetes mellitus type 2, Chronic Obstructive Pulmonary Disease(COPD), asthma, Crohn's disease, collitus, different types of cancer, Alzheimer's disease

Since its discovery in 1984, a lot of research has been dedicated to pro-inflammatory cytokines, in particular to the tumor necrosis factor. For example, an overview of the TNFα and Its activity is given in a review article by David J. MacEwan in TNF receptor subtype signalling: Differences and cellular consequences in Cell Signalling, 14 (2002), pages 477-482.

The interest in pro-inflammatory cytokines, in particular in TNFα Is understandable, since many of the diseases associated with TNFα are abundant and ever-spreading and the understanding of the mechanism behind the action of TNFα may help to find effective treatments of the diseases mentioned above.

TNFα is a ligand for cytokine receptors and as such the diseases associated with TNFα are diseases that are also associated with increased or decreased cytokine pathway activity.

The present invention provides an in vitro method for finding active pharmaceutical ingredients for the prevention, treatment or co-treatment of diseases associated with increased or decreased cytokine pathway activity.

In particular, the invention provides a method for finding an active pharmaceutical ingredient suitable for the treatment of a disease associated with increased or decreased cytokine pathway activity, comprising the steps of:
A1) providing a first lipid profile by
   a) providing a first cell culture medium comprising a first type of cells,
   b) stimulating the first type of cells, causing the cells to release a pro-inflammatory cytokine,
   c) bringing a second type of cells having cytokine receptors in a second cell culture medium into contact with the first cell culture medium comprising the first type of cells causing the cytokine receptors of the second type of cells to respond to the pro-Inflammatory cytokine that is released to obtain a cell culture medium that is in contact with the first and the second type of cells,
   d) adding a component that is potentially suitable for the prevention, treatment or co-treatment of a disease associated with increased or decreased cytokine pathway activity to the first cell culture medium and/or to the second cell culture medium and/or to the cell culture medium that is in contact with the first and the second type of cells,
   e) determining the lipid profiles of the second type of cells and/or of the first type of cells and/or of the cell culture medium that is in contact with the first and the second type of cells to obtain the first lipid profile,
A2) providing a second lipid profile by
   performing step A1) except for sub-step d) to obtain the second lipid profile,
B) comparing the first lipid profile to the second lipid profile and
C) identifying the component that was added in the method that shows a difference between the first lipid profile and the second lipid profile as the active pharmaceutical ingredient.

The method may further comprise the steps of:
A3) providing a third lipid profile by performing step A1) except for sub-step b) to obtain the third lipid profile,
A4) providing a fourth lipid profile by performing step A1) except for sub-steps b) and d) to obtain the fourth lipid profile,
D) comparing the third lipid profile to the fourth lipid profile and
E) identifying the component that was added in the method that shows a difference between the third lipid profile and the fourth lipid profile as the active pharmaceutical ingredient.

It has been found that the in vitro method of the invention Is capable of a (more) reliable prediction of the therapeutical effect of the active pharmaceutical ingredient thus identified In vivo (than any of the other methods known so far). The reason for this is that there is a continuous interaction between the production of pro-inflammatory cytokines such as TNFα in the first type of cells and the response of the second type of cells, whereas methods known so far look at the response of only one cell type.

Not only is the method of the invention capable of identifying active pharmaceutical ingredients suitable for treatment of acute diseases associated with Increased or decreased cytokine pathway activity, but may also be capable of identifying active pharmaceutical ingredients (APIs) that are especially suitable for treatment chronic diseases.

Examples of such chronic diseases include chronic inflammation, diabetes mellitus type 2, cancer, immune response associated diseases, (osteo)arthritis etc.

The term active pharmaceutical ingredient means any compound or mixture of compounds that has a prophylactic or therapeutic effect in vivo, preferably In a human.

Examples of diseases that are associated with Increased or decreased cytokine receptor activity Include but are not limited to those cited above, for example inflammation diseases, acute inflammation, chronic inflammation, psoriasis, perlodontitis, rheumatoid arthritis, cardiovascular diseases, osteoarthritis; atherosclerosis, asthma, septic shock, irritable bowel syndrome (IBS), heamorrhagic fever, cachexia, diabetes mellitus type 2, COPD, asthma, Crohn's disease, collitus, different types of cancer and Alzheimer's disease

In the method of the invention, the first type of cells is stimulated to cause the cells to release pro-inflammatory cytokines, preferably TNFα. Such stimulation can be done using methods known to the person skilled In the art.

The cells preferably release the pro-inflammatory cytokine into the cell culture medium in which they are present; this is also called secretion.

Examples of methods to stimulate the release of pro-inflammatory cytokines Include but are not limited to addition of a pro-inflammatory cytokine stimulating component, for example a lipopolysaccharide (LPS), such as the commercially available LPS from Sigma (productnumber L2630) or phorbol 12-mytistate 13-acetate (PMA), such as the commercially available PMA from Sigma (productnumber P8139) to the cell culture medium of the first type of cells.

The amount of pro-inflammatory cytokine stimulating component, for example lipopolysaccharide to be used is in principle not critical, but generally lies between 0.01 and 100µg/ml depending on the density of the cells.

Any combination of a first type of cells and a second type of cells may be used In the method of the present Invention. Animal cells are suitable, preferably mammalian cells. Particularly preferred are human cells. Other suitable cells include bacteria, cells of a fungus, yeast, algae and/or a plant The cells may be genetically modified or altered cells. Preferred combinations of cells include combinations where at least one type of cells is human cells, such as human (primary) cells and bacteria cells. Examples of (commercially) available cells include animal cells, for example NSO cells, CHO-cells and human cells, for example HeLa cells or PER.C6® cells.

Preferred examples of the types of cells that can be used in the method of the present invention include human primary cells, such as adipocytes / preadipocytes, bladder cells, brain cells, cardiac cells, endothelial cells, epidermal cells, epithelial cells, eye cells, fibroblasts, granulocytes, hepatic cells, human mesothelial cells, intestine and colon cells, lung and pulmonary cells, lymph cells, lymphocytes, mononuclear cells, neural nerve cells, phagocytes, renal cells, reproductive cells, skeletal cells, smooth muscle cells and umbilical cells. Further preferred examples of the types of cells that can be used in the method of the present invention include human stem cells such as mesenchymal stem cells, pericytes, CD34+ progenitor cells, CD133+ progenitor Cells, mononuclear cells and CD14+ Monocytes.

Preferably, the first type of cells are primary human peripheral blood mono-nuclear cells (PBMC) and/or the second type of cells are human aorta endothelial cells (HAOEC) . HAOEC may be commercially obtained from Cell Applications Inc. productnumber 304-05a.

The density of the first type of cells and of the second type of cells is in principle not critical and may for example be from 100,000 to 50,000,000 cells/ml or from 500 to 10,000,000 per cm².

The cell density as used herein may be determined using methods known to the person skilled in the art, for example by using a flow cytometer to count the number of cells in a given volume or on a given surface.

In the method of the invention further types of cells may be used in addition to the first and the second types of cells. For example in step b) of the method of the present invention, more than one type of cells may be stimulated to cause the release of pro-Inflammatory cytokines, for example TNFα and/or for example, more than one type of cells may be brought into contact in step c) of the method with the first cell culture medium.

The first type of cells and the second type of cells may be in direct contact with one another, but are preferably physically separated.

An example of a situation wherein the first type of cells and the second type of calls are in direct contact is when the first cell culture medium comprising a suspension of the first type of cells is contacted with a suspension of the second type of cells. An example of a situation wherein the first type of cells and the second type are physically separated include contacting of the second type of cells with the first cell culture medium wherein a membrane separates the second type of cells from the first type of cells.

A preferred way of physically separating the first type of cells and the second type of cells is to use a device which comprises
a first element with the first type of cells and a second element with the second type of cells, wherein the first type of cells may be present on a first surface of the first element and/or the second type of cells may be present on a second surface of the second element.

A preferred way of physically separating the first type of cells and the second type of cells is to use a device which comprises
a first element having a first surface on which the first type of cells is present and a second element having a second surface on which the second type of cells is present and wherein the first surface and the second surface are separated only by the cell culture medium.

A full description of said device can be found in the European patent application filed on 2 February 2011 with the European Patent Office, which patent application has received the following filing number 11000811.7. The content of this patent application is hereby Included in its entirety by reference.

In this device, the first surface and the second surface are therefore present In the same cell culture medium.

Such device makes the use of a membrane to get physical separation of the cells no longer necessary, whereas signalling molecules, such as pro-inflammatory cytokines can travel freely without being restricted by any membrane.

The fact that the first and the second surface are separated results in that the first and the second types of cells do not come in physical contact with each other, preventing the first and the second types of cells from attaching to each other. This avoids the risk of the first and the second types of cells influencing the secretion of signaling molecules by the presence of the first (second) type of cells next to the second (first) type of cells.

By using such device in the method of the invention, it is therefore possible to get a reliable prediction of the In vivo effect of the component that is potentially suitable for the prevention and/or treatment of a disease associated with Increased or decreased cytokine pathway activity.

Preferably, in the device, the first and the second surface are part of two detachable elements (hereafter also referred to as 'first element and second element'). This has an Important advantage of allowing the two type of cells to be removed from the respective surface independently In separate environments after the culturing for any further measurements. In the case where the first and the second surface are not detachable, removing one type of cells by e.g. use of trypsin would usually result In the removal of the other type of cells, resulting in a mixture of two different types of cells. This is unsuitable when analysis of one specific type of cells is desired, e.g. when the amount of a certain molecule in the specific type of cells needs to be measured. Furthermore, the device allows different types of cells to be subjected to different treatments in their respective cell culture media.

The chamber used in the device may be formed by any suitable material and may have any desired configuration. For example, the chamber may be in the form of a dish, a centrifuge tube, a cell culture tube, or the like. Exemplary materials for the chamber include, without limitation, glass, ceramic, polycarbonate, vinyl, polyvinyl chloride (PVC), polydimethylsiloxane (PDMS), acrylic, polypropylene, polyethylene, polyethyl sulfone (PES), and metal.

The first type of cells respectively the second type of cells may be present on the first respectively the second element by gravity only or by attachment of the cells to the surface.

In case gravity is used to ensure that cells are on their surface, care should be taken to keep the device in a steady position. This ensures that the cells are maintained in their respective positions. In other cases, the device according to the present invention may be subjected to shaking during the method of the invention over part or the whole of the method. This may facilitate the interaction between the different types of cells by Inducing more movement of signalling molecules such as pro-inflammatory cytokines, for example TNFα.

In a special form of the device, the first element may be a chamber containing the cell culture medium and the first surface may be the bottom of the chamber. The first type of cells then remains at the bottom of the first chamber by gravity. In most cases movement (e.g. by diffusion) of the first type of cells is small so that it will not reach the second type of cells and make a physical contact therewith. In the cases where it is desired to ensure no contact between the first and the second type of cells, the first type of cells at the bottom of the first chamber may be attached to the bottom. The second element may then be suspended in the culture medium so that the second surface is in the cell culture medium, i.e. under the surface of the culture medium. The second type of cells Is then preferably attached to the second surface of the second element. This is necessary In case the second type of cells Is present on the surface facing the bottom of the chamber. This is advantageous in that the first surface and the second surface face each other which makes it easy for the first (respectively second) type of signaling molecules to reach the second (respectively first) type of cells. In the cases where the second surface faces up, i.e. where the second type of cells rests on a surface of the second element facing up, it is not necessary that the second type of cells is attached to the second surface of the second element.

The suspended second element may take any suitable shape as long as it can be inserted in the first chamber. Further, suspending of the second element in the culture medium may be Implemented in a number of ways. For example, the suspended second element may e.g. be in a form of another chamber smaller than the chamber comprising the first type of cells at the bottom. The chamber to be inserted may have a rim that rests on the rim of the larger, receiving chamber or the suspended element may be a plate having a size that allows it to rest on the rim of the chamber. The plate may be provided with a protrusion protruding towards the bottom of the chamber, wherein the protrusion comprises the second surface to which the second type of cells is attached.

The suspended second element may also be implemented as a plurality of separate objects, e.g. in the form of bars or plates. The second type of cells may be attached to the surface of the bars or may rest on the plates. Where the suspended element comprises a plurality of separate or separable surfaces, this has an advantage that each of the surfaces may be removed from the culture medium at different time points for analysis so that time-dependent results can be obtained. The cells cultured In the same cell culture medium in the same way but attached to different surfaces may also be subjected to different analysis methods.

In other forms of the device, both the first and the second element may be suspended in the culture medium. The suspended element may take any suitable form, for example as described above In relation to the second element.

The first type of cells and the second type of cells are preferably attached to the first surface and the second surface, respectively.

It will be appreciated that only the surface on which the cells are attached needs to be in the culture medium and not the whole surface of the first and/or second element

The cells may be attached to the first and/or second surface in any known way. For example, collagen may be applied first and the cells may be subsequently applied. Another example is by providing a mechanical microstructure which is adapted so that cells can be attached thereto. Examples of such a mechanical microstructure of the surface include a textured microstructure and a porous microstructure having pores for accommodating the cells. Examples of ways of attaching cells to a surface are described e.g. in WO2007/106868. The description of WO2007/106868 is incorporated herein by reference with respect to the ways of attaching reagents to a membrane, which can be used In the present invention for attaching cells to the first and the second surfaces of the first and the second elements.

In case, more than two different types of cells are used, the first respectively second type of cells may be present on the first respectively second surface together with further different types of cells, i.e. two or more different types of cells may be present on the same surface.

The device used may be device as described above, wherein the second element Is a chamber containing a cell culture medium inserted in the chamber of the first element and the second surface comprises a membrane. Samples may be taken from the cell culture medium In the chamber of the second element for analysis. Accordingly, the method in these embodiments further comprises the step of subjecting the cell culture medium in the first chamber and/or the cell culture medium in the second chamber to an analysis while the method of the invention Is performed.

According to a further aspect of the Invention, a system is provided comprising a plurality of the device according to the present Invention. Preferably, the first element of each of the devices is detachably connected to each other and/or the second elements are detachably connected to each other. In one embodiment of this system, a first substrate is provided comprising a plurality of holes each of which acts as the chamber for comprising the culture medium and a second substrate is provided comprising a plurality of protrusions each of which can be inserted to each of the holes of the first substrate. Each of the pairs consisting of the hole and the protrusion acts in the same way as the device of the present invention. Preferably, each of the protrusions can be detached from the substrate. This facilitates (cells on) each of the protrusions to be subjected to analysis at different times. Similarly, if each of the holes can be detached from the substrate, subjecting (cells in) each of the holes to analysis at different times will become easier.

The method of the invention can in principle be performed in any type of cell culture medium suitable for the culturing of (animal) cells. Suitable cell culture media support the growth and differentiation of the first type of cells, the second type of cells and any further cells used In the method of the invention. Guidelines for choosing a cell culture medium and cell culture conditions are well known and are for instance provided in Chapter 8 and 9 of Freshney, R. I. Culture of animal cells (a manual of basic techniques), 4th edition 2000, Wiley-Liss and in Doyle, A., Griffiths, J. B., Newell, D. G. Cell & Tissue culture: Laboratory Procedures 1993, John Wiley & Sons.

Generally, a cell culture medium for (mammalian) cells comprises salts, amino acids, vitamins, lipids, detergents, buffers, growth factors, hormones, cytokines, trace elements and carbohydrates. Examples of salts Include magnesium salts, for example MgCl₂.6H₂O, MgSO₄ and MgSO₄.7H₂O iron salts, for example FeSO₄.7H₂O, potassium salts, for example KH₂PO₄, KCl; sodium salts, for example NaH₂PO₄, Na₂HPO₄ and calcium salts, for example CaCl₂.2H₂O. Examples of amino acids are all 20 known proteinogenic amino acids, for example hystidine, glutamine, threonine, serine, methionine. Examples of vitamins include: ascorbate, biotin, choline.Cl, myo-inositol, D-panthothenate, riboflavin. Examples of lipids Include: fatty acids, for example linoleic acid and oleic acid; soy peptone and ethanol amine. Examples of detergents include Tween 80 and Pluronic F68. An example of a buffer is HEPES. Examples of growth factors/hormones/cytokines include IGF, hydrocortisone and (recombinant) Insulin. Examples of trace elements are known to the person skilled In the art and include Zn, Mg and Se. Examples of carbohydrates Include glucose, fructose, galactose and pyruvate.

The culture medium may be supplemented with growth factors, metabolites, etc.

An example of a cell culture medium that may suitably be used for the first and/or second type of cells is RPMI (commercially available from Sigma) supplemented with 10% Foetal Calf Serum(FCS).

The optimal conditions under which the first type of cells; the first type of cells and the second type of cells; the first type of cells and the second type of cells and optionally the further type(s) of cells are cultured can easily be determined by the skilled person. For example, the pH, temperature, dissolved oxygen concentration and osmolarity of the cell culture medium are in principle not critical and depend on the type of cell chosen. Preferably, the pH, temperature, dissolved oxygen concentration and osmolarity are chosen such that these conditions optimal for the growth and productivity of the cells. The person skilled in the art knows how to find the optimal pH, temperature, dissolved oxygen concentration and osmolarity. Usually, the optimal pH is between 6.6 and 7.6, the optimal temperature between 30 and 39°C, for example a temperature from 36 to 38°C, preferably a temperature of about 37°C; the optimal osmolarity between 260 and 400mOsm/kg.

The duration of step b) of the method of the invention is in principle not critical as long as the cells are allowed the time to release the pro-inflammatory cytokines, for example TNFα. The time needed for the release of the pro-inflammatory cytokines, for example TNFα depends on the type of cells chosen and on way the cells are stimulated, such as for example the concentration of a component used to stimulate the release of the pro-inflammatory cytokines, for example TNFα. Generally, the duration of step b) is a matter of hours, for example a duration from 1 to 24 hours. Preferably, after stimulation of the first type of cells by adding a component to the first cells culture medium with a pro-inflammatory cytokine stimulating component, the cell culture medium is replaced by a fresh cell culture medium which does not contain the pro-Inflammatory cytokine stimulating component.

The presence of pro-inflammatory cytokines can be determined using methods known per se. For example, the presence of TNFα may be determined by flow cytometry making use of human TNFα FlowCytoMix from eBiosciences (productnumber 8223FF).

Examples of pro-inflammatory cytokines that may be released include but are not limited to IL-1b, IL-18, IL-4, IL-10, prostaglandins, for example PGE2, TNFα, preferably TNFα.

The component that is potentially suitable for the treatment of a disease associated with Increased or decreased cytokine pathway activity may be any component, for example a component from a library of chemical compounds. It may also be a mixture of compounds. The component may for example be a chemically synthesized compound, such as a steroid, but may also be a biological, such as a peptide, (monoclonal) antibody, siRNA, protein etc. or a compound isolated from a natural, for example from a plant.

The amount of component that is potentially suitable for the treatment of a disease associated with increased or decreased cytokine pathway activity may be anywhere in the range from 1.10⁻¹²mol/ml to 1 mol/ml. To enhance the response, the amount of said component may be increased step-wise using routine experimentation.

The component that is potentially suitable for the prevention, treatment or co-treatment of a disease associated with increased or decreased cytokine pathway activity may be added to the first cell culture medium and/or to the second cell culture medium and/or to the cell culture medium that is in contact with the first and the second type of cells. To which medium or media the component that is potentially suitable for the prevention, treatment or co-treatment of a disease associated with Increased or decreased cytokine pathway activity depends on the suspected activity of said component. If the component is suspected to neutralize the action of the pro-inflammatory cytokine (for TNFα this component would have suspected anti-TNFa action), the component is preferably added to the first cell culture medium and more preferably to both the first and second cell culture medium. If the component is suspected to be an antagonist or agonist for the cytokine receptor, the component is preferably added to at least the second cell culture medium or the cell culture medium that is in contact with the first and second type of cells. If the component is suspected to Interfere downstream of the cytokine receptor in the cytokine pathway, the component Is preferably added at least to the cell culture medium that is in contact with the first and the second type of cells.

In many cases however, it will be unclear what activity the component that Is potentially suitable for the prevention, treatment or co-treatment of a disease associated with increased or decreased cytokine pathway activity might have. In those cases, it is preferred to use the method of the invention multiple times to cover all possible combinations of to which medium or media the component is added.

The component that is potentially suitable for the prevention, treatment or co-treatment of a disease associated with increased or decreased cytokine pathway activity may be added to the cell culture medium before or after the second type of cells are brought Into contact with the cell culture medium of the first type of cells. If said component is added before the second type of cells are brought into contact with the cell culture medium of the first type of cells, the duration of the contact between the cells and said components is In principle not critical and may vary from for example 20 seconds to 24 hours. Also, the cell culture medium of the first or second type of cells to which the component that is potentially suitable for the prevention, treatment or co-treatment of a disease associated with increased or decreased cytokine pathway activity may be replaced by a cell culture medium not containing said component or containing said component in a lower amount before contacting the second type of cells with the cell culture medium of the first type of cells.

The lipid profiles may be determined using any suitable analysis method, which are known to the person skilled in the art. For example, analysis methods like flow cytometry, enzyme-linked immunosorbent assay (ELISA), liquid chromatography-mass spectrometry (LCMS), gas chromatography-mass spectrometry (GCMS) and immunocytochemistry may be used.

Preferably for the determination of the lipid profiles GCMS or LCMS are used.

The determination of the lipid profile may be done on the cells. If the analysis is done on the cells, the method of the invention may further comprise the steps of harvesting the cells by separation of the cells and the cell culture medium and optional disruption of the cell wall (prokaryotes) or cell membrane (eukaryotes) using methods known In the art. Alternatively, or also, the determination of the lipid profile may be done on the cell culture medium. In the cases where the cells are attached to a surface, before determining the lipid profile of the cells, the cells may be removed by use of e.g. trypsin. Alternatively, the cells may be directly lysed without removal from the surface using a lysis buffer (e.g. RIPA buffer as for example commercially available from Cellsignal).

The lipid profiles are preferably determined at different points in time (i.e. monitored), for example the lipid profiles may be determined at the start of the method of the invention (t = 0), after 1; 3; 6; 9; 12; 24; 48 and after 72 hours to get a good overview on the change of lipid profile.

It is noted that the method of the invention may be performed more than once at the same time and that the determining of the lipid profiles may be done at different points In time for the different simultaneously performed methods in order to ensure that the determination of the lipid profile by e.g. taking samples of the cell culture medium or of the cells does not change the result of measurements later in time.

With lipid profile is meant the amounts of lipids found in one sample. The lipid profile may Include the amounts of lipids chosen from individuals comprised in the groups of lysophospholipids, monoacylglycerols, phospholipids, sphingomyelines, diacylglycerols, cholesterol esters, ceramldes, for example a ceramide that is a sphingosine with fatty acid chains of from 16 to 24 C-atoms; triacylglycerols, sphingosine and sphingosine-1-phosphate.

The second lipid profile may be provided by a method comprising the steps of
a) providing a first cell culture medium comprising a first type of cells,
b) stimulating the first type of cells, causing the cells to release a pro-inflammatory cytokine, for example tumor necrosis factor α (TNFα)
c) bringing the second type of cells having cytokine receptors in a second cell culture medium into contact with the cell culture medium of the first type of cells causing the cytokine receptors of the second type of cells to respond to the pro-inflammatory cytokine, for example TNFα that may or may not be released by the first type of cells to obtain a cell culture medium that is in contact with the first and the second type of cells ande) determining the lipid profile of the second type of cells and/or of the first type of cells and/or of the cell culture medium that is in contact with the first and the second type of cells to obtain the second lipid profile and
e) determining the lipid profile of the second type of cells and/or of the first type of cells and/or of the cell culture medium that is in contact with the first and the second type of cells to obtain the second lipid profile, wherein the method does not include the step of
d) adding a component that Is potentially suitable for the prevention, treatment or co-treatment of a disease associated with increased or decreased cytokine pathway activity to the first cell culture medium and/or to the second cell culture medium and/or to the cell culture medium that is In contact with the first and the second type of cells.

The first and the second lipid profiles are lipid profiles of the cells and/or the cell culture medium obtained by method including step b), i.e. it is a lipid profile of the cells and/or the cell culture medium in which the first type of cells are stimulated to release a pro-inflammatory cytokine.

The second lipid profile is a lipid profile of the cells and/or the cell culture medium obtained by a method not including step d), i.e. It Is a lipid profile of the cells and/or the cell culture medium to which a component that is potentially suitable for the prevention, treatment or co-treatment of a disease associated with increased or decreased cytokine pathway activity is not added. Thus, comparison between the first lipid profile and the second lipid profile allows determining the effect of the component that is potentially suitable for the prevention, treatment or co-treatment of a disease associated with increased or decreased cytokine pathway activity when the first type of cells are stimulated to release a pro-inflammatory cytokine.

The third lipid profile may be provided by a method comprising the steps of
a) providing a first cell culture medium comprising a first type of cells,
c) bringing the second type of cells having cytokine receptors in a second cell culture medium into contact with the cell culture medium of the first type of cells causing the cyrokine receptors of the second type of cells to respond to the pro-inflammatory cytokine, for example TNFα, that may or may not be released by the first type of cells to obtain a cell culture medium that Is In contact with the first and the second type of cells,
d) adding a component that is potentially suitable for the prevention, treatment or co-treatment of a disease associated with increased or decreased cytokine pathway activity to the first cell culture medium and/or to the second cell culture medium and/or to the cell culture medium that is in contact with the first and the second type of cells and
e) determining the lipid profile of the second type of cells and/or of the first type of cells and/or of the cell culture medium that is in contact with the first and the second type of cells to obtain the third lipid profile, wherein the method does not include the step of b) stimulating the first type of cells, causing the cells to release a pro-inflammatory cytokine.

The fourth lipid profile may be provided by a method comprising the steps of
a) providing a first cell culture medium comprising a first type of cells,
c) bringing the second type of cells having cytokine receptors In a second cell culture medium Into contact with the cell culture medium of the first type of cells causing the cytokine receptors of the second type of cells to respond to the pro-inflammatory cytokine, for example TNFα, that may or may not be released by the first type of cells to obtain a cell culture medium that is in contact with the first and the second type of cells,
e) determining the lipid profile of the second type of cells and/or of the first type of cells and/or of the cell culture medium that Is in contact with the first and the second type of cells to obtain the third lipid profile, wherein the method does not include the steps of b) stimulating the first type of cells, causing the cells to release a pro-inflammatory cytokine and
d) adding a component that is potentially suitable for the prevention, treatment or co-treatment of a disease associated with increased or decreased cytokine pathway activity to the first cell culture medium and/or to the second cell culture medium and/or to the cell culture medium that is in contact with the first and the second type of cells.

The third and the fourth lipid profiles are lipid profiles of the cells and/or the cell culture medium obtained by method not including step b), i.e. it is a lipid profile of the cells and/or the cell culture medium In which the first type of cells are not stimulated. The difference between the third and the fourth lipid profiles Is that a component that Is potentially suitable for the prevention, treatment or co-treatment of a disease associated with increased or decreased cytokine pathway activity is added for obtaining the third profile. Thus, comparison between the third lipid profile and the fourth lipid profile allows determining the effect of the component that is potentially suitable for the prevention, treatment or co-treatment of a disease associated with increased or decreased cytokine pathway activity when the first type of cells are not stimulated.

Thus, the component which induces a change between the first lipid profile and the second lipid profile and/or a change between the third lipid profile and the fourth lipid profile is identified to be an active pharmaceutical ingredient.

It will be appreciated that the steps for providing the lipid profiles is not critical may be performed in any order. For example, the first lipid profile may be provided after the second lipid profile, or the fourth lipid profile may be provided before any other lipid profiles are provided.

The first lipid profile may also be compared to the third or the fourth lipid profile.

Preferably, also further lipid profiles are determined, for example a fifth lipid profile may be provided by repeating sub-steps a), b) and e) of the method to determine the fifth lipid profile.

In particular, the fifth lipid profile may be provided by a method comprising the steps of
- providing a first cell culture medium comprising a first type of cells,
- stimulating the first type of cells, causing the cells to release a pro-inflammatory cytokine and
- determining the lipid profile of the first type of cells to obtain the fifth lipid profile.

For example, a sixth lipid profile may be provided by a method comprising the steps of
- providing the first type of cells in the first cell culture medium and
- determining the lipid profile of the first type of cells to obtain the sixth lipid profile.

For example, a seventh lipid profile may be provided by a method comprising the steps of
- providing the second type of cells in the second cell culture medium and
- determining the lipid profile of the second type of cells to obtain the seventh lipid profile.

For example, an eighth lipid profile may be provided by a method comprising the steps of
- stimulating the second cells in the second cell culture medium the same way as the first cells were stimulated and
- determining the lipid profile of the second type of cells to obtain the eighth lipid profile.

For obtaining the lipid profiles, lipid profile of the cells, the cell culture medium or both may be determined.

The first lipid profile obtained in the method of the invention is compared to the second lipid profile, but may also be compared to any further lipid profile or combinations of any further lipid profiles, for example it may be compared to the third lipid profile, fourth lipid profile, the fifth lipid profile, the sixth lipid profile, the seventh lipid profile and/or the eighth lipid profile which can be obtained as described above.

Therefore, the invention also relates to a method of the invention further comprising the step of providing a fifth lipid profile by
repeating steps a), b) and e) of the method to determine the first lipid profile to obtain the fifth lipid profile and
- also comparing the first lipid profile to the fifth lipid profile
- and identifying the component that was added in the method that shows a difference between the first lipid profile and the second lipid profile and the fifth lipid profile as the active pharmaceutical ingredient

The invention also relates to a method of the invention further comprising the step of providing a six lipid profile by a method comprising the steps of
- providing the first type of cells in the first cell culture medium and
- determining the lipid profile of the first type of cells to obtain the sixth lipid profile.

The invention also relates to a method of the invention further comprising the step of providing a seventh lipid profile by a method comprising the steps of
- providing the second type of cells In the second cell culture medium and
- determining the lipid profile of the second type of cells to obtain the seventh lipid profile.

The Invention also relates to a method of the invention further comprising the step of providing a seventh lipid profile by a method comprising the steps of
- stimulating the second cells in the second cell culture medium the same way as the first cells were stimulated and
- determining the lipid profile of the second type of cells to obtain the eighth lipid profile.

Preferably, the lipids of which the lipid profiles are compared are chosen from the group of ceramldes, for example a ceramide that Is a sphingosine with fatty acid chains of from 16 to 24 C-atoms, sphingosine and sphingosine-1-phosphate.

The differences in lipid profiles between the first, second and optionally any further lipid profile (such as the third, fourth, fifth, sixth, seventh, eighth lipid profile as described above) determined should be assessed in methods that make use of the same cells, conditions etc. Similarly, the differences between the third and the fourth profiles should be assessed in methods that make use of the same cells, conditions etc. Also, the differences should be assessed at the same point in time. For example, if a difference can only be seen in step e) after 6 hours after adding the component that is potentially suitable for the prevention, treatment or co-treatment of a disease associated with increased or decreased cytokine pathway activity, then this difference should be noted and the component identified as active pharmaceutical ingredient.

In another aspect, the invention relates to a composition comprising the active pharmaceutical ingredient obtained with the method of the invention, wherein the composition Is a pharmaceutical composition further comprising a pharmaceutically acceptable carrier.

A person skilled in the art knows which carriers may be used as pharmaceutically acceptable carriers. Examples of such pharmaceutically acceptable carriers are both inorganic and organic carrier materials, suitable for oral/parenteral/injectable administration and include water, gelatin, gum arabic, lactose, starch, magnesium stearate, talc, vegetable oils, and the like.

Besides the active pharmaceutical ingredient and a pharmaceutically acceptable carrier, the pharmaceutical composition according to the present invention, may further comprise conventional pharmaceutical additives and adjuvants, excipients or diluents, including, but not limited to, water, gelatin of any origin, vegetable gums, ligninsulfonate, talc, sugars, starch, gum Arabic, vegetable oils, polyalkylene glycols, flavoring agents, preservatives, stabilizers, emulsifying agents, buffers, lubricants, colorants, wetting agents, fillers, and the like.

In another aspect, the invention relates to an active pharmaceutical ingredient obtained with the method of the invention or to the pharmaceutical composition of the invention for use as a medicament.

In yet another aspect, the invention relates to an active pharmaceutical ingredient obtained with the method of the Invention or to the pharmaceutical composition of the invention for use In the prevention, treatment, or co-treatment of a disease associated with increased or decreased cytokine pathway activity.

Examples of diseases associated with increased or decreased cytokine pathway activity are given herein.

In yet another aspect, the invention relates to use of an active pharmaceutical ingredient obtained with the method of the invention or to the pharmaceutical composition of the invention for the manufacture of a medicament for the prevention, co-treatment or treatment of a diseases associated with increased or decreased cytokine pathway activity.

In another aspect, the invention relates to a method for treatment, co-treatment or prevention of a disease associated with increased or decreased cytokine pathway receptor activity In animals, including humans, said method comprising the step of administering an effective amount of the active pharmaceutical ingredient obtained with the method of the invention or administering an effective amount of the pharmaceutical composition of the invention to animals including humans which are in need thereof.

With 'effective amount' of the active pharmaceutical Ingredient obtained with the method of the invention or of the pharmaceutical composition of the invention is meant the amount needed to treat, co-treat or prevent the disease associated with Increased or decreased cytokine pathway receptor activity in animals, including humans.

In the framework of the Invention, with animals Is meant all animals, including mammals, examples of which include humans. Preferred examples of mammals beside humans include dogs, dromedarls, camels, elephants, and horses.

Although the invention has been described in detail for purposes of illustration, it is understood that such detail is solely for that purpose, and variations can be made therein by those skilled in the art without departing from the spirit and scope of the invention as defined in the claims.

It is further noted that the invention relates to all possible combinations of features described herein, especially those combinations of features that are present in the claims.

The invention will now be illustrated with the following examples without however being limited thereto.

### Examples

### Fixing cells on the inserting chamber

A cylindrical cup for Inserting in another cup was provided (hereinafter sometimes referred as an inserting chamber). The inserting chamber had a rim which can rest on another cup when inserted and had a membrane as the bottom. The inserting chamber was commercially available from Greiner Blo-One as ThinCert (TM). The membrane was of PET and had pores of a diameter of 0.4 µm with a pore density of 2,000,000 per cm².

The inserting chamber was placed upside down, i.e. the inserting chamber was placed so that the inside of the Inserting chambers were facing downward.

500 µL of collagen coating solution (from CellApplications, catnr. 125-50) was placed on the outer surface of the membrane of the Inserting chamber using a pipet. The entire outer surface of the membrane was covered and allowed to stand for 4 hours at room temperature in a flow cabinet. Subsequently, the collagen coating solution was washed away from the membrane with PBS. The Inserting chambers were stored at 4 °C until use.

A suspension of human aorta endothelial cells (HAOEC) from Cell Applications Incorporated containing 50,000 cells/ml was prepared in endothelial growth medium from Cell Applications Incorporated. 1 ml of the cell suspension was coated on the outer surface of the membrane coated with the collagen coating solution. The inserting chamber was left for 2 hours in a flow cabinet to allow the cells to firmly attach to the outer surface of the membrane via the collagen. Subsequently, the cells were brought into contact with endothelial growth medium from Cell Applications incorporated and allowed to culture for 24 hours.

A total of three inserting chambers having HAOEC attached on the outer surface of the membrane were made by the method described above, with 90% confluence.

### Providing cells in the receiving chamber

Three chambers each having a larger diameter and depth (hereinafter sometimes referred as a receiving chamber) than the inserting chamber was provided.

Primary human peripheral blood mono-nuclear cells (PBMC) were seeded at the bottom of the receiving chamber comprising a culture medium (RPMI1640 from Sigma) at 1,000,000 cells/chamber. The PBMC were stimulated with 10 µg/ml lipid poly saccharide (LPS). After 1 hour the culture medium was replaced by the same culture medium without LPS and PBMC were left to secrete TNF-α for another 6 hours.

### Co-culturingof cells

The inserting chambers with HAOEC were inserted In the receiving chambers with LPS stimulated PBMC. The distance between the surface with HAOEC and the surface with PBMC was 0.2 mm. 3 ml of a culture medium of RPMI 1640 was added to each of the receiving chambers and 1 ml of the same culture medium was added to each of the inserting chambers. The three pairs of chambers were placed In an incubator at a temperature of 37°C and having an atmosphere of 5% O₂ and 95% CO₂ and cells were cultured.

At timepoints 0,1, 3, 6, 9, 12, and 24 (48 and 72) hours samples of culture medium from the insert, culture medium from the well, the PBMC cells and the HAoEC cells were taken and frozen at -80°C until measurements (TNFα, cyclooxygenase 2 (COX2), PGE2 and lipid profiles) were executed.

TNFα was determined by flow cytometry using Human TNF-alpha FlowCytomix from eBiosciences (productnr : 8223FF) according to the instructions of the supplier.

COX2 and PGE2 were measured by Elisa using COX2 Elisa kit CBA053 from Calbiochem and Prostaglandin E2 EIA Kit, Ref. 514010 from Cayman.

Lipid profiles were determined by means of the following LCMS method:

To a cell suspension of 100.000 HAoEC In 100 µl, 10 ul Internal standard (STD) (C₁₇ ceramide) and 750µl MeOH/CHCl_{δ} (2/1) (MeOH stands for methanol) were added and this was vigorously shaken for 10 minutes. Subsequently, the mixture was incubated at 48°C for 1 hour. Then 75 µl 1N KOH In MeOH were added and mixed. This mixture was incubated at 37°C. After 1 hour the pH was adjusted to pH - 5 by addition of 10% acetic acid.

Then, 750 µl of CHCl₃ were added and mix with the sample. Samples were centrifuged at 2600 rpm at 4°C for 3mlnutes. The organic phase was taken and reduced to dryness. After sonication for 3 minutes and centrifuging at 800g for 10 minutes the residue was reconstituted in a eluent mixture of 80% B and 20% A (see below).

***LC-Conditions:*** Gradient elution according to the following table:

| | |
|---|---|
| *Eluent A* | 5 mM ammonium acetate In methanol/H₂O (1:1) containing 1% acetic acid and 0.25% tetrahydrofurane |
| *Eluent B* | 5 mM ammonium acetate in methanol containing 1% acetic acid and 0.25% tetrahydrofuran |
| | |
| *Gradient* | 0-0.1 min 1% B isocratic, In 1.9 min to 100% B and stays at 100% B for 6 min. Total analysis time is 12,5 minutes. The flow rate is 700 µL/min. |
| *Column* | 2.1 x 100 mm, 1.8 µm Zorbax SB-C18-or equivalent - operating at 50°C. |
| *Injection* | Injection volume: 1-2 µL. |

### MS Ionisation conditions:

ESI(+), MRM mode, fragmentor optimized for each compound (120 V); EMV +400 V, 300°C gas temperature, flow 5 L N₂/min, 45 psi, sheath gas temperature 250 °C, sheath gas flow 10 L N₂/min, 3.5 kV cap, 0.5 kV nozzle, dwell time 5 ms. Two time segments are used; 0-2.5 minutes for sphingosine and sphingosine-1-phosphate and 2.5-12.5 minutes for the ceramides.

**MS *MRM conditions***: tandem MS in the MRM mode:
Time segment 0-2.5 min:
   Sphingosine; 300.2 → 282,2; EC 5 V
   Sphingosine-1-phosphate; 380.1 → 264.2; EC 20 V
Time segment 2.5-12.5 min:
   C₁₂; 482.4 → 264.2; EC 15 V
   C₁₅; 538.4 → 264.2; EC 20 V
   C₁₇ (IS); 552.0 → 264.2; EC 20 V
   C_{18:1}; 564.4 → 264.2; EC 20 V
   C₂₀; 594.5 → 264.2; EC 20 V
   C₂₂; 622.5 → 264.2; EC 20 V
   C_{24:1}; 648.5 → 264.2; EC 20 V
   C₂₄; 650.6 → 264.2; EC 25 V

The results of this experiment are shown in figures 1 to 5.

Fig. 1 describes the concentration of TNFα in the cell culture medium of the receiving chamber (with the PBMC cells) In time under three different conditions. The conditions mentioned are as follows:
EPL stands HAoEC cells which were contacted with LPS stimulated PBMC cells.
PL stands for LPS stimulated PBMC cells.
P stands for PBMC cells (which were not stimulated).

Fig. 2 describes the expression of cyclooxygenase-2 (COX-2) in the HAoEC cells in time under three different conditions.

The conditions mentioned are as follows:
EC stands for unstimulated HAoEC cells.
EPL stands HAoEC cells which were contacted with LPS stimulated PBMC cells.
EP stands for HAoEC cells which were contacted with non-stimulated PBMC cells

Fig. 3 describes the PGE2 release in the culture medium of the receiving chamber (with the PBMC cells) in time under four different conditions.

The conditions mentioned are as follows:
EC stands for unstimulated HAoEC cells
EPL stands HAoEC cells which were contacted with LPS stimulated PBMC cells
EP stands for HAoEC cells which were contacted with non-stimulated PBMC cells
PL stands for LPS stimulated PBMC cells.

Fig. 4 describes the lipid profile of the HAoEC cells of an experiment wherein HAoEC cells were contacted with LPS stimulated PBMC cells (EPL) in time; it specifically describes the concentrations of several ceramides: sphingomyeline with fatty acid side chains of 16 (C16), 18 (C18), 20 (C20), 22 (C22) or 24 (C24) C-atoms In time. In case of the sphingomyeline with fatty acid side chains of 24 C-atoms a sphingomyeline with an unsaturated fatty acid side chain (C24:1) and a sphingomyeline with a saturated fatty acid side chain (C24) were measured.

Fig. 5 describes the lipid profile of endothelial cells in time; It specifically describes the concentrations of several ceramldes: sphingomyeline with fatty acid side chains of 16 (C16), 18 (C18), 20 (C20), 22 (C22) or 24 (C24) C-atoms in time. In case of the sphingomyeline with fatty acid side chains of 24 C-atoms a sphingomyeline with an unsaturated fatty acid side chain (C24:1) and a sphingomyeline with a saturated fatty acid side chain were measured.

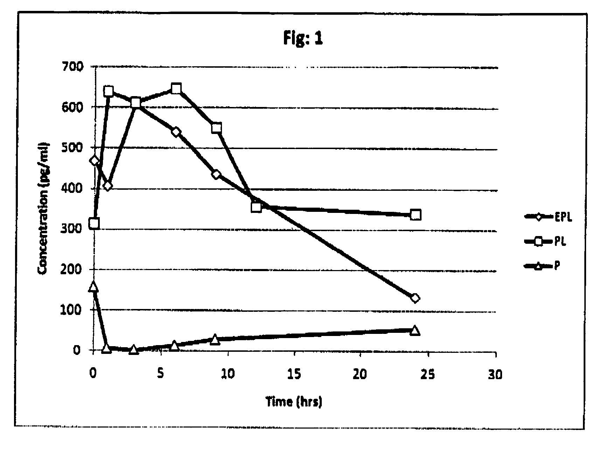

As can be seen from Fig. 1, stimulation by LPS of the PBMC cells leads to release of the pro-inflammatory cytokine TNFα. As can also be seen, the presence of HAoEC (EPL experiment) leads to a faster decrease of the TNFα than the case wherein HAoEC cells are not present (PL experiment), indicating an interaction between the TNFα released by the PBMC cells and the HAoEC cells.

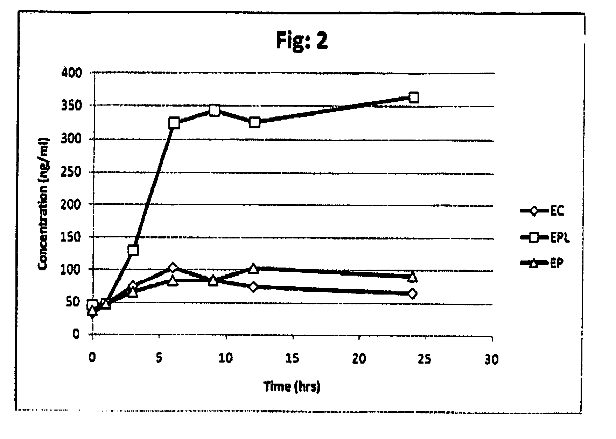

As can be seen from Fig. 2, COX-2 Is only expressed in HAoEC cells when PBMC cells are stimulated with LPS and contacted with HAoEC cells (EPL).

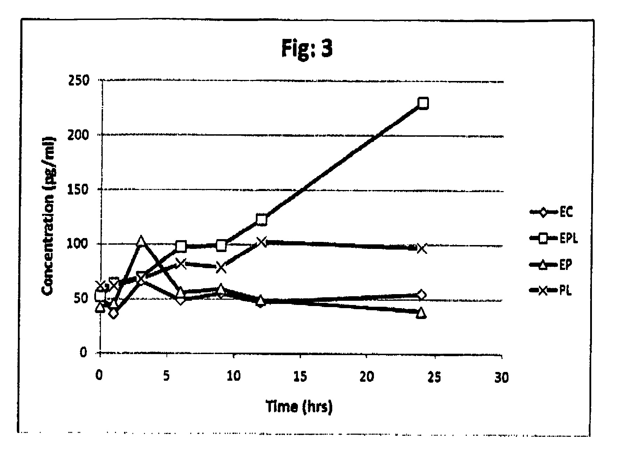

As can be seen from Fig. 3, the PGE2 concentration increases in case the PBMC cells (PL) are stimulated with LPS or when the HAoEC cells are contacted with LPS stimulated PBMC cells (EPL). The increase In case of stimulation of the HAoEC cells is significantly larger.

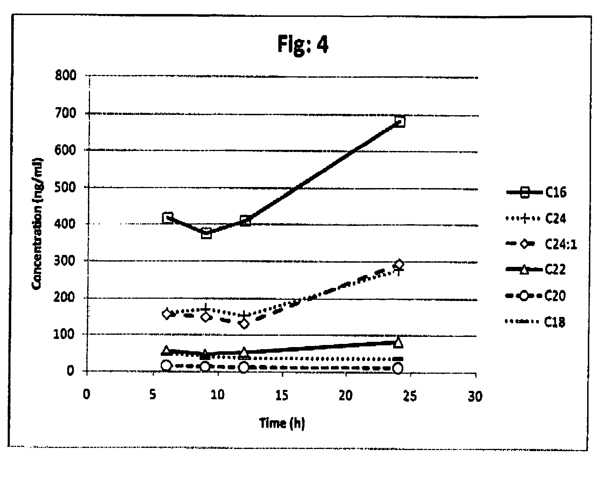

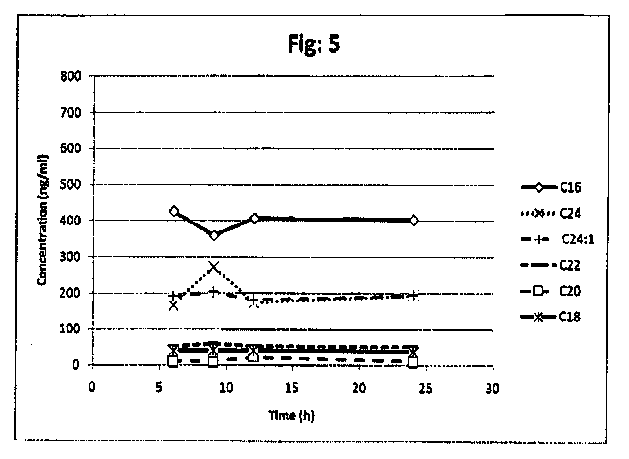

As can be seen by comparing Fig. 4 with Fig 5, by stimulating the PBMC cells, the lipid concentrations of the lipids determined In the HAoEC cells change in time.

## Claims

1. Method for finding an active pharmaceutical ingredient suitable for the treatment of a disease associated with Increased or decreased cytokine pathway activity, comprising the steps of
A1) providing a first lipid profile by
a) providing a first cell culture medium comprising a first type of cells,
b) stimulating the first type of cells, causing the cells to release a pro-inflammatory cytokine,
c) bringing a second type of cells having cytokine receptors in a second cell culture medium into contact with the cell culture medium of the first type of cells causing the cytokine receptors of the second type of cells to respond to the pro-inflammatory cytokine that is released to obtain a cell culture medium that is in contact with the first and the second type of cells
d) adding a component that is potentially suitable for the prevention, treatment or co-treatment of a disease associated with increased or decreased cytokine pathway activity to the first cell culture medium and/or to the second cell culture medium and/or to the cell culture medium that is In contact with the first and the second type of cells and
e) determining the lipid profiles of the second type of cells and/or of the first type of cells and/or of the cell culture medium that is in contact with the first and the second type of cells to obtain the first lipid profile,
A2) providing a second lipid profile by performing step A1) except for sub-step d) to obtain the second lipid profile,
B) comparing the first lipid profile to the second lipid profile and
C) identifying the component that was added in the method that shows a difference between the first lipid profile and the second lipid profile as the active pharmaceutical ingredient.

2. Method according to claim 1, further comprising the steps of:
A3) providing a third lipid profile by performing step A1) except for sub-step b) to obtain the third lipid profile,
A4) providing a fourth lipid profile by performing step A1) except for sub-steps b) and d) to obtain the fourth profile,
D) comparing the third lipid profile to the fourth lipid profile and
E) Identifying the component that was added in the method that shows a difference between the third lipid profile and the fourth lipid profile as the active pharmaceutical ingredient.

3. Method according to claim 1 or 2, wherein the pro-inflammatory cytokine is tumor necrosis factor α (TNFα).

4. Method according to claim 1, 2 or 3, wherein the first type of cells are primary human peripheral blood mono-nuclear cells (PBMC) and/or the second type of cells are human aorta endothelial cells (HAOEC).

5. Method according to any one of claims 1-4, wherein the first type of cells and the second type of cells are physically separated by using a device which comprises a first element with the first type of cells and a second element with the second type of cells, wherein the first type of cells may be present on a first surface of the first element and/or the second type of cells may be present on a second surface of the second element

6. Method according to claim 5, wherein the first and the second surface are part of two detachable elements.

7. Method according to claim 5 or claim 6, wherein the first type of cells and the second type of cells are attached to the first respectively to the second surface

8. Method according to any one of claims 1-7, further comprising the step of
A5) providing a fifth lipid profile by
repeating steps a), b) and e) of the method to determine the fifth lipid profile and
- also comparing the first lipid profile to the fifth lipid profile
- and identifying the component that was added In the method that shows a difference between the first lipid profile and the second and the fifth lipid profile as the active pharmaceutical Ingredient.

9. Method according to any one of claims 1-8, further comprising the step of providing an eighth lipid profile by a method comprising the steps of
- stimulating the second cells in the second cell culture medium the same way as the first cells were stimulated and
- determining the lipid profile of the second type of cells to obtain the eighth lipid

10. Method according to any one of claims 1-9, wherein the lipids of which the profiles are compared are chosen from the group of ceramldes, for example a ceramide that is a sphingosine with fatty acid chains of from 16 to 24 C-atoms, sphingosine and sphingosine-1-phosphate.

11. Composition comprising the active pharmaceutical ingredient obtained with the method of any one of claims 1-10, wherein the composition is a pharmaceutical composition further comprising a pharmaceutically acceptable carrier.

12. Active pharmaceutical ingredient obtained with the method of any one of claims 1-10, or composition according to claim 11 for use as a medicament.

13. Active pharmaceutical Ingredient obtained with the method of any one of claims 1-10, or composition according to claim 19 for use in the prevention, treatment or co-treatment of a disease associated with increased or decreased cytokine pathway activity.

14. Use of an active pharmaceutical ingredient obtained with the method of any one of claims 1-11, or composition according to claim 11 for the manufacture of a medicament for the prevention, co-treatment or treatment of a disease associated with increased or decreased cytokine pathway activity.

15. A method for treatment, co-treatment or prevention of a disease associated with increased or decreased cytokine pathway receptor activity in animals, including humans, said method comprising the step of administering an effective amount of the active pharmaceutical ingredient obtained with the method of any one of claims 1-10 or administering an effective amount of the pharmaceutical composition of claim 11 to animals including humans which are in need thereof.
